# EUROPEAN PATENT APPLICATION

(11) **EP 4 169 960 A1**
(43) Date of publication of application: **26.04.2023**
(21) Application number: 21829991.5
(22) Date of filing: 23.06.2021
(51) Int. Cl.: C08F 299/04, C08F 2/50, C08L 55/00, A61L 31/06, A61L 31/14, A61B 17/11, B29C 64/124

(54) **TEMPERATURE-DEPENDENT SHAPE MEMORY POLYMER**

(30) Priority: 23.06.2020 KR 20200076806
(71) Applicant: TMD Lab Co. Ltd, Seoul 04799 (KR)
(72) Inventor: PARK, Ju Young, Seoul 03722 (KR); KANG, Mi-Lan, Seoul 03722 (KR); YI, Se Won, Seoul 03722 (KR)
(74) Representative: J A Kemp LLP
(86) International application number: PCT/KR2021/007894
(87) International publication number: WO 2021/261915

(57) **Abstract**

The present invention relates to a shape memory polymer that can be restored to an original shape from a deformed shape by means of body temperature in the body. When using the shape memory polymer of the present invention and a device for wrapping the outer wall of blood vessels prepared thereby, it is possible to effectively prevent abnormal blood vessel dilatation, and prevent stenosis by effectively inhibiting neointimal formation.

## Description

### Technical Field

The present invention was made with the support of the Ministry of SEMs and Startups of the Republic of Korea under Project No. S2796595, which was executed in the research project named "Development of Shape Memory Polymer Tube for Lacrimal Duct Insertion for Treating Nasolacrimal Duct Obstruction" in the research program titled "Startup Growth-Technology Development Project" by the TMD Lab under the management of the Korea Technology and information Promotion Agency for SMEs, from 25 November 2019 to 24 November 2021.

This application claims priority to and the benefit of Korean Patent Application No. 10-2020-0076806 filed in the Korean Intellectual Property Office on 23 June 2020, the disclosure of which is incorporated herein by reference.

The present invention relates to a shape memory polymer capable of recovering to its original shape from its deformed form by the body temperature in the body.

### Background Art

Various shapes of stents for intravascular insertion have been conventionally developed and used. Most stents are used in the form of being inserted into blood vessels to prevent stenosis and occlusion. However, there has been a need for a structure capable of suppressing the dilation of the blood vessel by attachment to the external wall of the blood vessel in order to solve the problems resulting from the dilation of the external wall of the blood vessel. Specifically, for example, when an anastomosis is performed as needed to connect the artery and the vein, an excessive dilation may occur at a site of the vein connected with the artery due to a blood pressure difference between the artery and the vein, resulting in side effects, such as edema at the surgical region, and may block arterial and venous conduits of the surgical region in the long run. Therefore, there has been a need for a device for wrapping an outer vascular wall, the device capable of preventing an excessive dilatation and edema due to a blood pressure difference at a site of the vein connected to the artery and the vortex of the blood flow to inhibit clot formation and neointima formation.

Korean Patent No. 10-1906472 discloses a shape memory polymer in the art, and the present inventors established that a shape memory polymer technique used in the conventional art can be preferably applied to a device for wrapping an outer vascular wall, and attempted to derive a polymer compound having excellent properties as a structurally differentiated individual polymer compound considering conventionally disclosed shape memory polymers.

### Disclosure of Invention

### Technical Problem

The present inventors have made intensive research efforts to develop a biocompatible polymer structure capable of being preferably applied for outer vascular wall wrapping. As a result, the present inventors established that a 4-arm copolymer containing a central carbon atom with four carbon-carbon binding arms, when applied to the outer vascular wall, can provide flexible elasticity while effectively inhibiting abnormal dilation of the blood vessel and the resultant side effects, and thus can be preferably used for outer vascular wall wrapping, and completed the present invention.

Accordingly, an aspect of the present invention is to provide a shape memory polymer formed by crosslinking of a 4-arm structured copolymer.

Another aspect of the present invention is to provide a device for wrapping an outer vascular wall, the device being composed of the above-described shape memory polymer.

Still another aspect of the present invention is to provide a method for manufacturing a device for wrapping an outer vascular wall.

### Solution to Problem

In accordance with an aspect of the present invention, there is provided a shape memory polymer containing a compound represented by Chemical Formula 1 below: 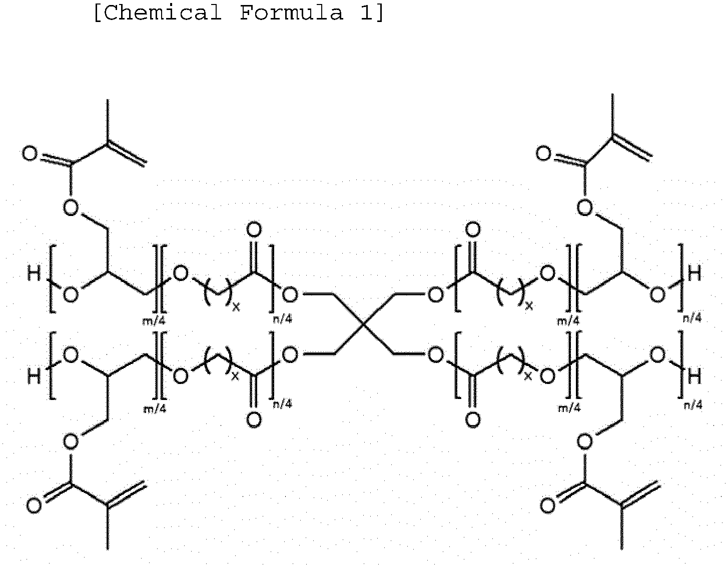 wherein,
x is an integer of 1 to 20;
m and n represent the mole % of repeating units, respectively; and
m + n is 100 and m is 80 to 96.

The present inventors established that a 4-arm copolymer containing a central carbon atom with four carbon-carbon binding arms, when applied to the outer vascular wall, can provide flexible elasticity while effectively inhibiting abnormal dilation of the blood vessel and the resultant side effects, and thus can be preferably used for outer vascular wall wrapping.

In an embodiment of the present invention, x is an integer of 2 to 10 in Chemical Formula 1. In another embodiment, x in Chemical Formula 1 may be an integer of 2 to 9, an integer of 2 to 8, an integer of 2 to 7, an integer of 2 to 6, an integer of 2 to 5, an integer of 3 to 10, an integer of 3 to 9, an integer of 3 to 8, an integer of 3 to 7, an integer of 3 to 6, an integer of 3 to 5, an integer of 4 to 10, an integer of 4 to 9, an integer of 4 to 8, an integer of 4 to 7, an integer of 4 to 6, an integer of 4 to 5, an integer of 5 to 10, an integer of 5 to 9, an integer of 5 to 8, an integer of 5 to 7, or an integer of 5 to 6. More specifically, a compound of Chemical Formula 1 in which x is 5 may be used, but is not limited thereto.

In an embodiment of the present invention, m in Chemical Formula 1 may be 85 to 96, and more specifically, for example, m may be 85 to 96, 88 to 96, 90 to 96, or 92 to 96.

In one embodiment of the present invention, the shape memory polymer according to the present invention may be a four-arm copolymer containing a central carbon atom having four carbon-carbon binding arms, as a copolymer of an ε-caprolactone monomer and glycidyl methacrylate.

According to another aspect of the present invention, there is provided a device for wrapping an outer vascular wall, the device containing the above-described shape memory polymer.

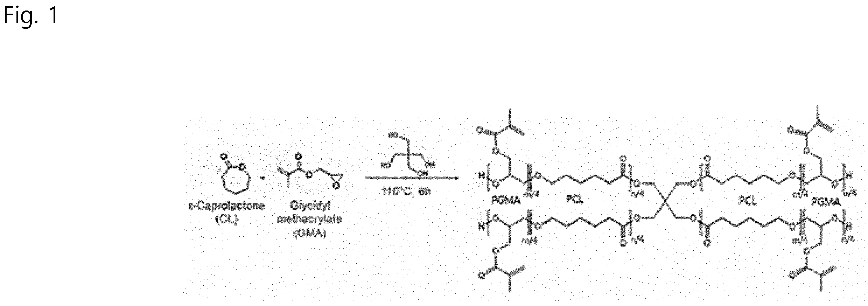

As a concept contrasting with the device for wrapping an outer vascular wall of the present invention, stents are well known designed in various practical forms to be inserted into blood vessels to prevent blood vessel obstruction. A porous device for wrapping an outer vascular wall of the present invention is designed to wrap the outer vascular wall from the outside of the blood vessel in the body, and may be fabricated to have an inside diameter (I.D.), an outer diameter (O.D.), a length, or the like, which is adjusted according to the type and diameter of the blood vessel to be treated, the purpose of medical procedure, or the like. A specific example is shown in FIG. 1.

For convenience, the term "porous device for wrapping an outer vascular wall" may be interchangeably used with "device for wrapping an outer vascular wall", "porous device for wrapping a blood vessel", "device for wrapping a blood vessel", "apparatus for wrapping a blood vessel" or the like.

In an embodiment of the present invention, the outer vascular wall to which the device for wrapping an outer vascular wall of the present invention is applied may be an outer vascular wall of a blood vessel anastomosis site. As for the blood vessel anastomosis site, a difference in characteristic between anastomosed blood vessels, especially a difference in pressure resistance to endure the pressure generated by the blood flow may cause the dilatation of the blood vessel at one side of the blood vessel. The device for wrapping an outer vascular wall according to an embodiment of the present invention can easily wrap the external wall of the anastomosis site and effectively solve the dilation of one side of the blood vessel.

In an embodiment of the present invention, the blood vessel anastomosis site of the present invention is an anastomosis site between two different blood vessels selected from a vein, an artery, and an artificial blood vessel.

In an embodiment of the present invention, the blood vessel anastomosis site of the present invention is a vein-artery anastomosis site.

In one embodiment of the present invention, the blood vessel anastomosis site of the present invention may be a vein-artificial blood vessel anastomosis site or an artificial blood vessel-artery anastomosis site. The blood vessel anastomosis between the artery and the vein may be made via an artificial blood vessel, and anastomosis may be made in the order of vein - artificial blood vessel - artery, wherein the device for wrapping an outer vascular wall of the present invention can be applied to the vein-artificial blood vessel anastomosis site or the artificial blood vessel-artery anastomosis site, and especially, may be preferably applied to the vein-artificial blood vessel anastomosis site.

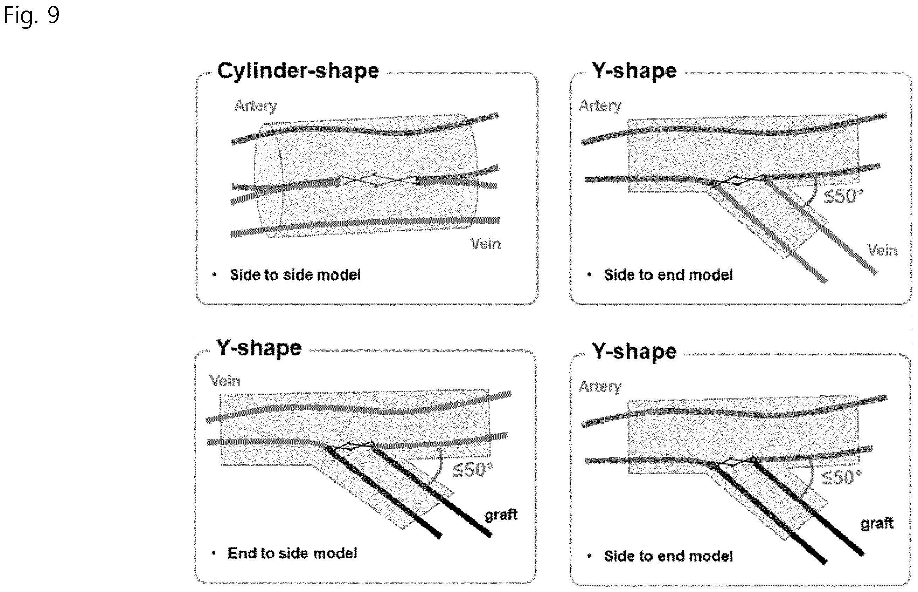

The anastomosis between heterogeneous blood vessels includes all of a "side to side model" of connecting sides of heterogeneous vessels to each other and an "end to side model" or "side to end model" of connecting one end of one blood vessel to one side of the other blood vessel. A cylindrical device for wrapping an outer vascular wall may be preferably used for the "side to side model", and a Y-shaped device for wrapping an outer vascular wall may be preferably used for the "end to side model" or "side to end model" (see FIG. 9).

The blood vessel anastomosis site in the present invention may be formed due to a surgery requiring a blood vessel junction, specifically, a surgery requiring a heterogeneous blood vessel junction, and more specifically, for example, arteriovenous fistula surgery, coronary artery bypass surgery, or the like, and the device for wrapping an outer vascular wall of the present invention may be applied to the formed blood vessel anastomosis site.

In an embodiment of the present invention, the device for wrapping an outer vascular wall of the present invention has an original shape or a temporary shape, wherein the original shape is in a hollow cylindrical tube shape or a hollow Y-tube shape, one side of which is cut in a lengthwise direction to enable a blood vessel to be inserted into a hollow space, wherein the temporary shape is maintainable at room temperature and is in a curved surface shape or a planar surface shape with both ends formed by the cutting being separated from each other, and wherein when applied to an outer vascular wall, the temporary shape is bent in a direction of increasing the curvature to wrap the outer vascular wall, thereby recovering to the original shape. More specifically, when the original shape is in a cylindrical tube shape, the cutting may be linear cutting that is formed in a length direction (axial direction) of the cylindrical tube shape, and when the original shape is in a Y-tube shape, the cutting may be a Y-shape cutting that is formed on one surface of the Y-tube shape.

As used herein, the term "original shape" refers to a shape of the device for wrapping an outer vascular wall of the present invention, which is formed and maintained in the in-vivo environment condition. Regarding the temperature condition as a specific example, the shape refers to a shape that is formed in the condition of, on average, 28°C to 42°C, including the body temperature. More specifically, the shape refers to a shape that is formed in the condition of 35°C to 38°C. The above-described "Y-tube shape" refers to a shape in which two cylindrical tube shape are connected in a Y-shape to communicate each other similarly to the external appearance of the blood vessel anastomosis site.

As used herein, the term "temporary shape" refers to a shape of the porous device for wrapping an outer vascular wall of the present invention, which is stably maintainable at room temperature, which is a typical storage condition, or at a temperature lower than room temperature, and more specifically, may be a state in which both ends formed by the above-described cutting are separated to open, for the application to the outer vascular wall. Still more specifically, the room temperature herein for describing the temporary shape means a temperature range of about 1°C to 28°C, and the temporary shape of the porous device for wrapping an outer vascular wall of the present invention may be stably maintained in not only a room temperature environment but also a lower temperature environment lower than room temperature, and specifically, for example, at a temperature of lower than 1°C. More specifically, for example, the temporary shape may be stably maintained even under a low temperature condition of lower than 1°C to - 78°C.

In an embodiment of the present invention, the device for wrapping an outer vascular wall of the present invention wraps a blood vessel and then maintains its recovered original shape in an in-vivo environment condition.

In accordance with another aspect of the present invention, there is provided a method for manufacturing a device for wrapping an outer vascular wall, the method including:
(a) photo-crosslinking a mixture of the above-described shape memory polymer and a photo-initiator to a hollow cylindrical tube shape or a hollow Y-tube shape to prepare a tube-type device;
(b) cutting one side of the tube-type device in a length direction parallel to the central axis of a hollow space formed in the cylindrical tube or Y-tube; and
(c) inducing the tube-type device with one side cut, into a temporary shape allowing the insertion of a blood vessel under a temperature condition exceeding the body temperature and then fixing the tube-type device by cooling at a temperature lower than room temperature.

A method for manufacturing a device for wrapping an outer vascular wall according to an aspect of the present invention can be used to produce a temperature-sensitive shape memory device for wrapping an outer vascular wall. More specifically, the device for wrapping a blood vessel, produced by the manufacturing method of the present invention, may be deformed to a temporary shape, described in another aspect of the present invention, in a temperature condition (lower than 28°C) lower than room temperature and a temperature condition (42°C or higher) higher than the body temperature (about 36.5-37°C) and then recovered to its original shape in the vivo-like environment condition. More specifically the device for wrapping a blood vessel, produced by the manufacturing method of the present invention, may be deformed to a temporary shape, described in another aspect of the present invention, in a temperature condition (42°C or higher) higher than the body temperature (about 36.5-37°C) and then recovered to its original shape under in the vivo-like environment condition.

A manufacturing method according to an embodiment of the present invention will be described in detail by steps.

### Step (a) : photo-crosslinking mixture of shape memory polymer and photo-initiator to hollow cylindrical tube or Y-tube shape to prepare tube-type device

As used herein, the term "photo-initiator" refers to a substance that can initiate and/or accelerate photo-crosslinking of a polymer and, specifically, refers collectively to a substance that is added to, for example, a composition to be UV- or LED-crosslinked and cured to thereby absorb energy from a UV or LED light source, thereby initiating the crosslinking of the polymer. To implement the present invention, various known photoinitiators may be used without limitations. More specifically, 1,5,7-triazabicyclo[4.4.0]dec-5-ene (TBD), tin(II) (2-ethylhexanoate), trimethylopropane tris(3-mercaptopropionate), zinc succinate, or phenylbis(2,4,6-trimethylbenzoyl)phosphine oxide (Igacure), which commercially usable, may be used but is not limited thereto.

To perform this step, the above-described shape memory polymer may be used by being dispersed in a content of 40 wt% to 60 wt%, more specifically, about 50 wt%, in a generally used, commercially available solvent, for example, dichloromethane, and the photo-initiator may also be used by being dispersed in a content of 5 wt% to 15 wt%, more specifically, about 10 wt%, in a generally used, commercially available solvent, for example, dichloromethane, wherein the dispersion solutions may be used by mixing at a volume ratio of 15:1 to 5:1, more specifically, 12:1 to 8:1, and still more specifically, 10:1. The prepared mixture solution and the porogen may be mixed at a weight ratio (w/w%) of 1:2 to 2:1, and still more specifically about 1:1, thereby preparing a final mixture.

The final mixture thus prepared may be fixed in a cylindrical tube shape or Y-tube shape before being cured. To fix the final mixture in the form of a cylindrical tube shape or Y-tube shape, the final mixture may be spray injected into an empty space in the form of a cylindrical tube or Y-tube shape in a mold. As a specific example, a mold prepared by inserting a silicone tube into a Teflon tube may be used as the mold with an empty space in the form of a cylindrical tube or Y-tube shape, but is not limited thereto. In addition, crosslinking may be performed in a UV or LED crosslinking machine. The time for photo-curing may be appropriately adjusted depending on characteristics of the photo-initiator, and any commercially available UV or LED curing lamp system may be used without limitations.

In an embodiment of the present invention, the mixture of the shape memory polymer and the photo-initiator in step (a) of the present invention may be further mixed with a porogen.

As used herein, the term "porogen" refers to a substance that is used to form pores in a cured polymer structure. The porogen may be removed after the curing of the polymer, thereby obtaining a polymer structure in which pores are formed corresponding to portions into which the porogen is incorporated.

In an embodiment of the present invention, the above-described porogen may be at least one selected from the group consisting of gelatin, sodium chloride, sodium bicarbonate, ammonium bicarbonate, polyethylene glycol, and hexane. Specifically, for example, when gelatin is used as the porogen of the present invention, the shape memory polymer of the present invention is mixed and crosslinked with gelatin together with the photo-initiator during crosslinking by UV or LED, and then treated in water at 40°C for 24 hours or longer, thereby dissolving and removing gelatin from the cured polymer structure. When sodium chloride, sodium bicarbonate, ammonium bicarbonate, polyethylene glycol, hexane, or the like is used as the porogen of the present invention, the corresponding porogen may be dissolved and removed by the same method as in gelatin. After the removal of the porogen is finished, the cured polymer structure may be separated from the mold and may be subjected to sufficient washing.

In an embodiment of the present invention, the photo-crosslinking in step (a) of the present invention may be performed such that pores are formed by a 3D printer.

In an embodiment of the present invention, the 3D printer of the present invention may be driven in any one manner selected from stereo lithography apparatus (SLA), digital light processing (DLP), and photopolymer jetting technology (PolyJet), but is not limited thereto.

As described above, the tube-type device prepared in step (a) of the present invention may be prepared by using (i) a porogen incorporation-removal manner or (ii) a manner of molding into a shape containing pores, specifically a manner of performing 3D printing in a shape containing pores. When the above-described porogen incorporation-removal manner (i) is used, the removal of the porogen may be performed before, after, or simultaneously with cutting in step (b).

### Step (b) cutting one side of tube- or Y-type device in length direction parallel to central axis of hollow space formed in cylindrical tube or Y-tube shape

As described above, a step of cutting one side of the tube-type device in a length direction parallel to the central axis of the hollow space formed in the cylindrical tube or Y-tube shape may be performed before or after the removal of the porogen or after 3D printing. The term "cutting" refers to a procedure of forming a "cutting line", along which one side of the cylindrical tube or Y-tube shape is cut to enable a blood vessel to be inserted into the hollow space of the tube-type device. Specifically, for example, for a cylindrical tube shape, a linear cutting line may be formed in a length direction parallel to the central axis of the hollow space formed, and for a Y-tube shape, two linear cutting lines may be formed at a cross-point thereof in length directions parallel to the central axes of two respective communicating cylindrical tube shapes, thereby forming a cutting line similar to the letter "y".

### Step (c): Inducing tube-type device with one side cut, into temporary shape allowing insertion of blood vessel under temperature condition exceeding body temperature and then fixing tube-type device by cooling at temperature lower than room temperature

In an embodiment of the present invention, "temperature condition exceeding body temperature" may be a temperature condition of 42°C or higher, 43°C or higher, 44°C or higher, 45°C or higher, 46°C or higher, 47°C or higher, 48°C or higher, 49°C or higher, or 50°C or higher, more specifically, 42°C to 65°C, still more specifically 45°C to 65°C, and still more specifically, 50°C to 60°C. The tube-type device becomes relatively easy to mold under the above temperature condition, wherein the device may be molded into a predetermined temporary shape by application of a physical force and then the temporary shape may be fixed at a temperature (1-28°C) lower than room temperature. For example, the temporary shape may be fixed by cooling at more preferably a sufficiently low temperature, and specifically a temperature for a dry ice condition (about -70°C). The temporary shape once fixed as such can maintain its temporary shape at room temperature or at a temperature lower than room temperature.

According to an embodiment of the present invention, the present invention may further include an additional porosity providing step after any one of steps (a) to (c). The additional porosity providing step of the present invention may be performed by punching the cured polymer structure, and specifically may be performed by, for example, biopsy punch. In still another embodiment, additional porosity may be provided by using a femto second laser light source technique or pores may be formed by using a mold capable of forming a porous structure. The porous structure formed by the above-described methods may have an average diameter of, for example, 100 um to 1000 um, 200 um to 700 um, or 300 um to 500 um. The porosity may be adjusted between 30 and 80% of the total area of a stent. In another embodiment, the porosity may be adjusted between 40 and 75% or 50 to 70%.

Since the manufacturing method of the present invention pertains to the method for manufacturing a "device for wrapping an outer vascular wall" according to another aspect of the present invention, the overlapping part recites the related content, and the overlapping description is omitted to solve the excessive complexity of the present description.

In accordance with still another aspect of the present invention, there is provided a device for wrapping an outer vascular wall produced by the above-described "method for manufacturing a porous device for wrapping an outer vascular wall" according to another aspect of the present invention.

### Advantageous Effects of Invention

Features and advantages of the present disclosure are summarized as follows.
(a) The present invention provides a shape memory polymer formed by crosslinking of a 4-arm structured copolymer.
(b) The present invention provides a device for wrapping an outer vascular wall, the device being composed of the above-described shape memory polymer.
(c) The present invention provides a method for manufacturing a device for wrapping a outer vascular wall.
(d) The shape memory polymer and the device for wrapping an outer vascular wall, manufactured of the shape memory polymer, of the present invention, can be used to effectively prevent abnormal blood vessel dilatation.
(e) The shape memory polymer and the device for wrapping an outer vascular wall, manufactured of the shape memory polymer, of the present invention, can be used to effectively inhibit neointima formation, thereby preventing stenosis.

### Brief Description of Drawings

FIG. 1 illustrates a schematic diagram of an example of synthesis of a 4-arm structured copolymer. The monomers CL and GMA were added together with pentaerythritol as an initiator and reacted at 110°C for 6 hours, thereby synthesizing 4-arm PCL-PGMA.

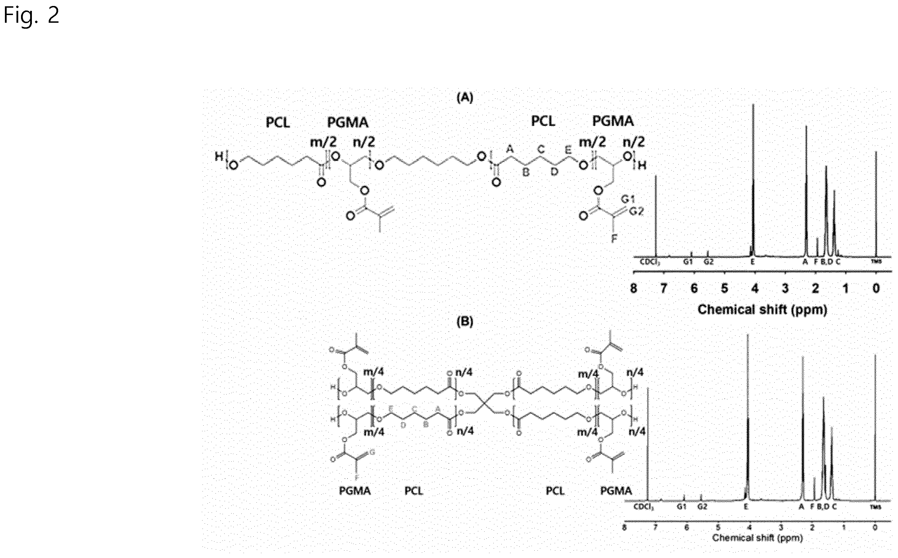

FIG. 2 illustrates NMR data for identifying a 4-arm PCL-PGMA copolymer as an example of a 4-arm structured copolymer. PCL peaks were confirmed at δ=4.10 [m, -OCH₂, (E)], 2.41 [m, -CH₂, (A)], 1.74 [m, -CH₂, (B and D)], and 1.45 [m, -CH₂, (C)], and PGMA peaks were confirmed at δ=6.13 [s, =CH₂, (G2)], 5.58 [s, =CH₂, (G1)], 1.97 [s, - CH₃, (F)], indicating that 4-arm PCL-PGMA was synthesized.

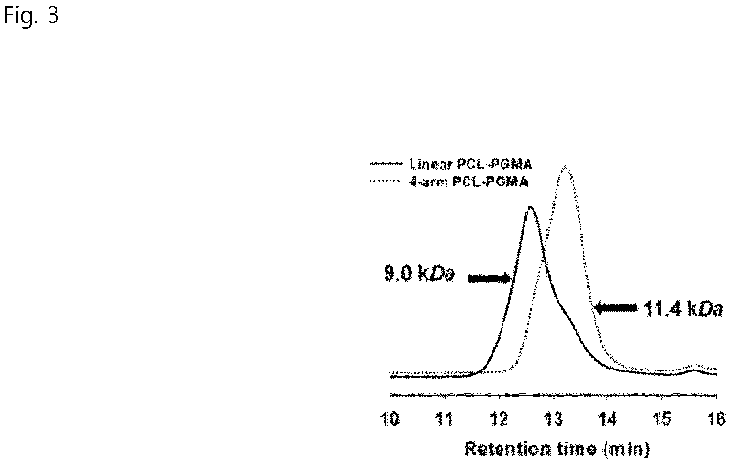

FIG. 3 illustrates a GPC measurement graph to determine the molecular weight of the synthesized polymer. The 4-arm polymer had a peak at a longer retention time than the linear polymer, and thus had a larger molecule weight.

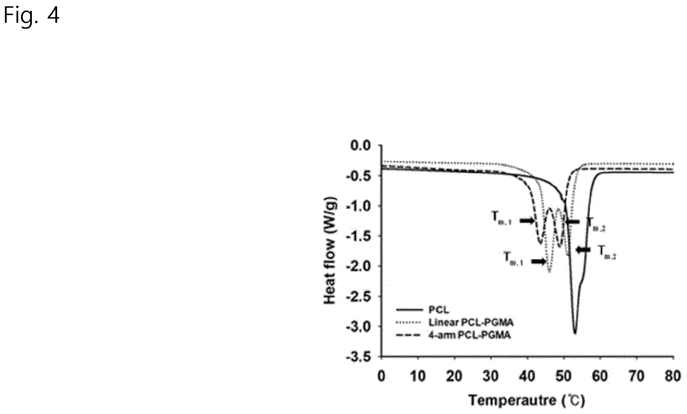

FIG. 4 illustrates a DSC measurement graph before crosslinking of the synthesized polymer. The reason that two peaks are shown in the linear PCL-PGMA (comparative example) and the 4-arm PCL-PGMA (example) compared with PCL is that the random binding of PGMA to PCL lowers the crystallinity of PCL to result in a difference in crystallinity. In addition, the peaks of the 4-arm PCL-PGMA are lower than those of the linear PCL-PGMA due to structural differences.

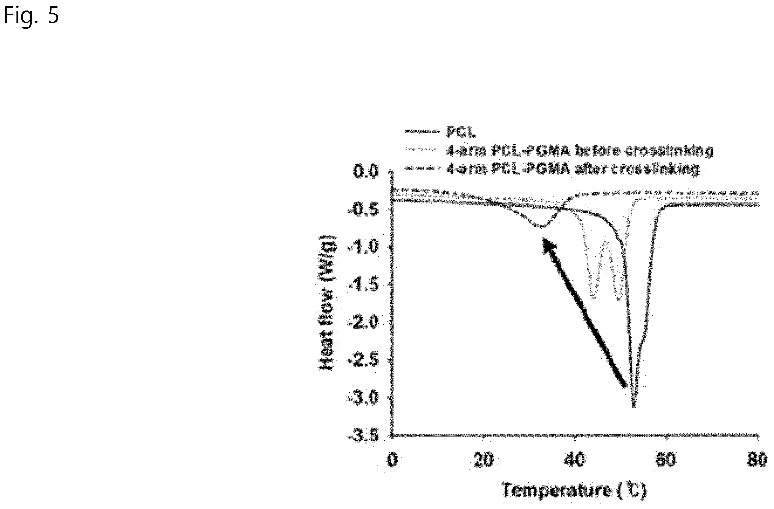

FIG. 5 illustrates a DSC graph before and after crosslinking of the example (4-arm PCL-PGMA). The random binding of PGMA to the comparative example (PCL) to lower crystallinity and thus lower the melting temperature of PCL, and the lowering of the crystallinity of PCL by PGMA makes a difference in crystallinity, thereby forming two peaks. After crosslinking, a network structure is formed by reaction of methacrylate, and thus one peak is shown and the melting temperature is lowered.

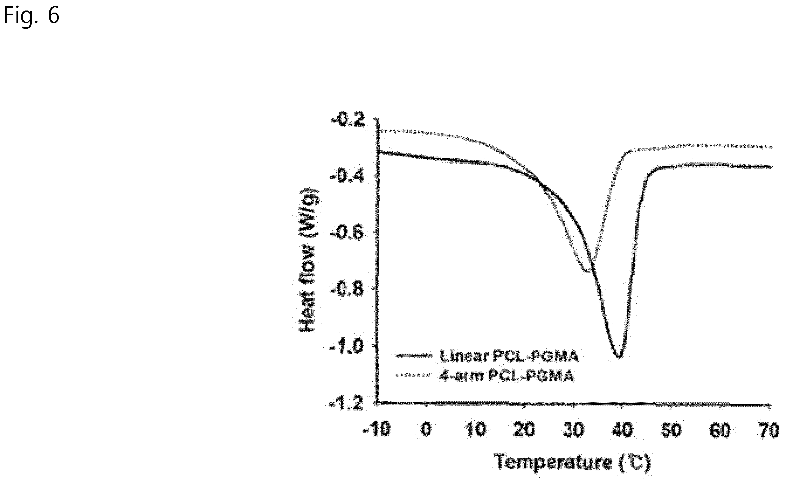

FIG. 6 illustrates a DSC graph after crosslinking of the linear PCL-PGMA (comparative example) and the 4-arm PCL-PGMA (example). As a result of comparing thermal characteristics of the two polymers, the melting temperature of the 4-arm polymer was lower. Therefore, the shape recovery was attained at a low temperature and the recovery rate to the original shape at the body temperature could be increased.

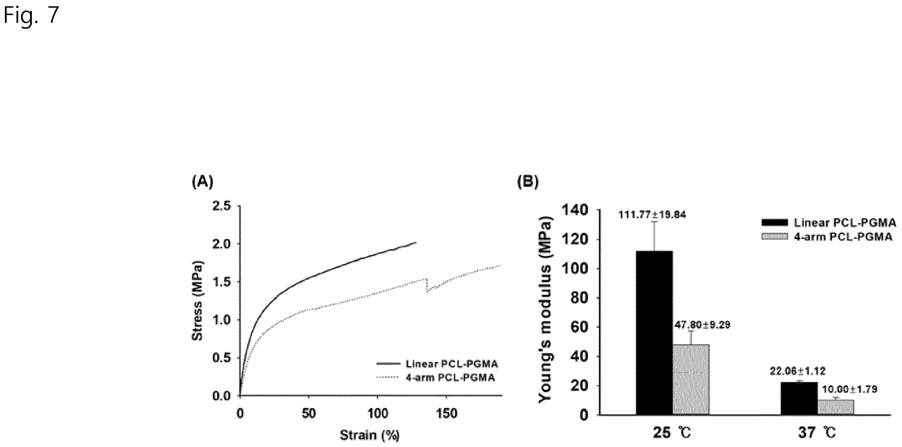

FIG. 7 illustrates tensile strength measurement graphs after polymer crosslinking. FIG. 7A shows strainstress curves of the comparative example (linear PCL-PGMA) and the example (4-arm PCL-PGMA) measured at the body temperature, 37°C; and FIG. 7B illustrates a graph comparing Young's modulus between the comparative example and the example at room temperature (25°C) and the body temperature (37°C). In the tensile strength measurement at 37°C, the example showed a higher elongation than the comparative example due to its rubbery properties (FIG. 7A). The example showed a lower Young's modulus at 25°C and 37°C than the comparative example and thus had an advantage of being rubbery, resulting in easier deformation at 25°C and 37°C, so that when inserted into a curved part in the body, the example was less bent to reduce a foreign-object sensation (FIG. 7B).

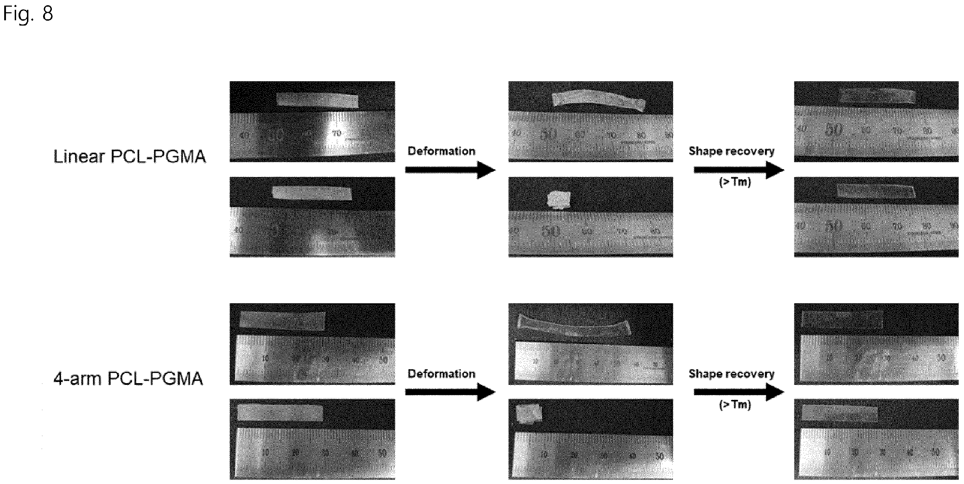

FIG. 8 illustrates images confirming the shape recovery ability of the 4-arm polymer of the present invention. The sample with an original shape was deformed by pulling or folding at 55°C or higher. The deformed sample was fixed with liquid nitrogen to be converted to a temporary shape, which was then subjected to heating at Tm value or higher to investigate the shape memory ability. The 4-arm PCL-PGMA was well recovered from the temporary shape to the original shape.
FIG. 9 illustrates schematic diagrams of cylinder shape and Y-shape devices for wrapping an outer vascular wall and schematic diagrams of the arrangement of an artery, a vein, and an artificial blood vessel (graft) at regions at which the respective types of devices were applied. The respective diagrams show: a cylinder shape device for wrapping an outer vascular wall, the device being applied to a side to side model for connecting a side of the artery and a side of the vein (top left); a Y-shape device for wrapping a outer vascular wall, the device being applied to a side to end model for connecting a side of the artery and an end of the vein (top right); when a side of the vein and a side of the artery are separately connected to ends of the artificial blood vessel, a Y-shape device for wrapping an outer vascular wall, the device being applied to an end to side model for connecting the side of the vein and the end of the artificial blood vessel (bottom left); and a Y-shape device for wrapping an outer vascular wall, the device being applied to an end to side model for connecting the side of the artery and the end of the artificial blood vessel (bottom right).

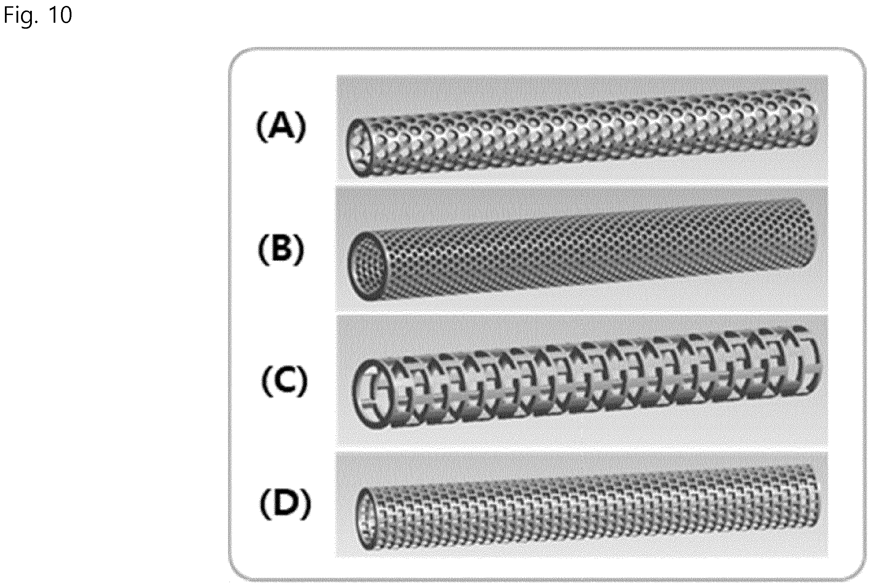

FIG. 10 illustrates manufacturing schematic diagrams through the control of the mesh type or size of a device for wrapping an outer vascular wall by using a 3D printer. The drawings show a large circular mesh structure (A), a small circular mesh structure (B), a large square mesh structure (C), and a small square mesh structure (D).

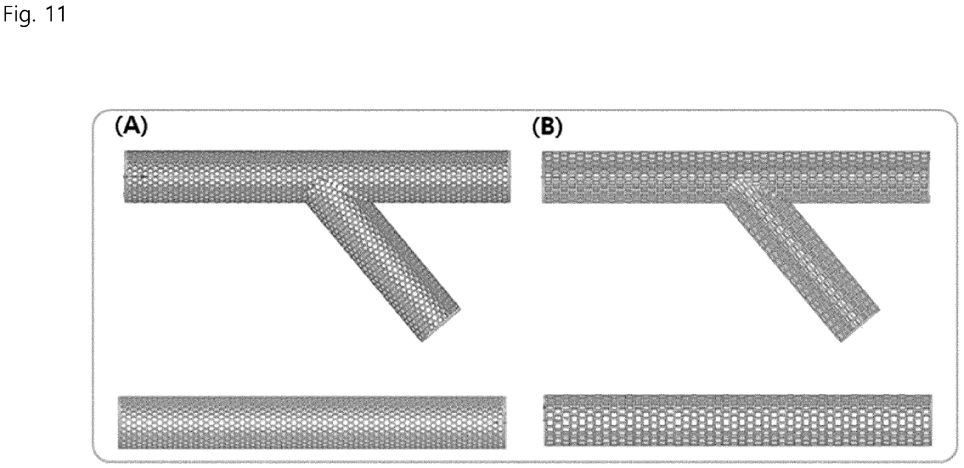

FIG. 11 illustrates designs for manufacturing devices for wrapping an outer vascular wall by using a 3D printer. The diagrams show a Y-shape and small circular mesh design (A, top), a cylinder shape and small circular mesh design (A, bottom), a Y shape and small square mesh design (B, top), and a cylinder shape small square mesh design (B, bottom).

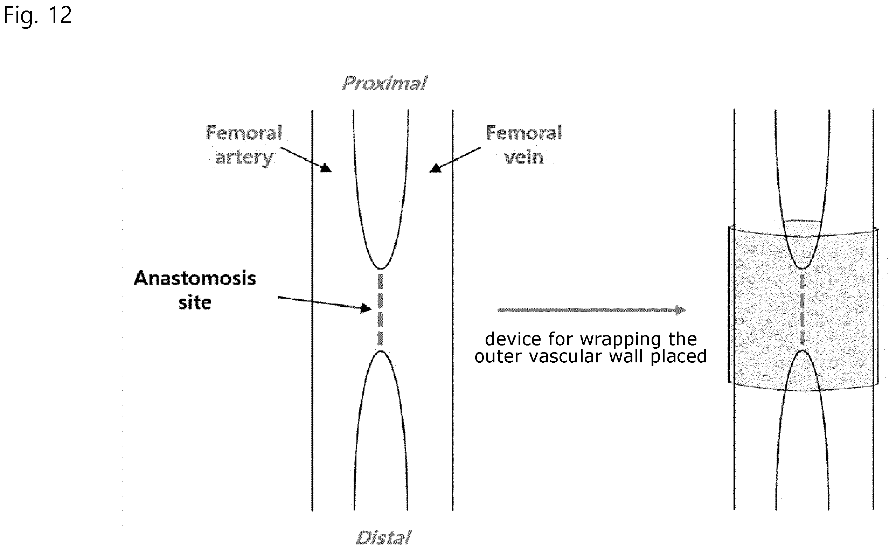

FIG. 12 illustrates schematic diagrams showing an arteriovenous fistula model (side-to-side) prepared using the femoral vein and the femoral artery of a beagle dog and a state in which a device for wrapping an outer vascular wall was placed on the arteriovenous fistula model.

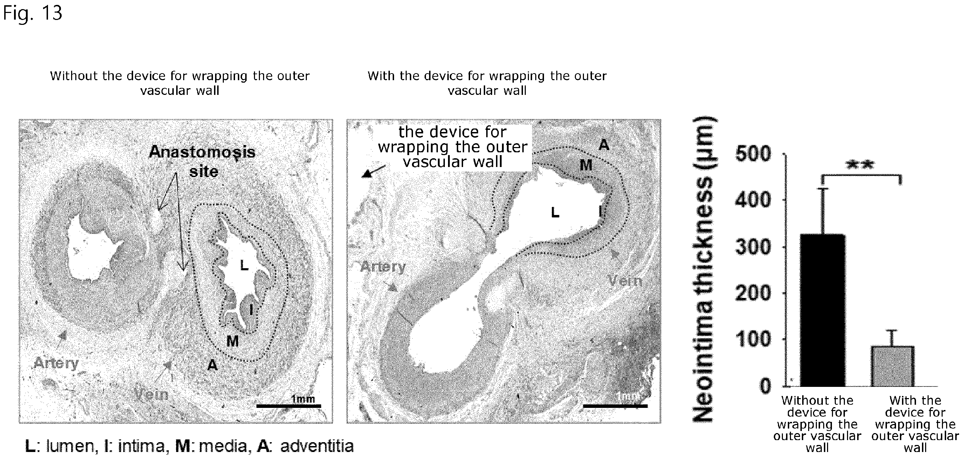

FIG. 13 illustrates histological analysis (H&E staining) results of a blood vessel anastomosis site on which a device for wrapping an outer vascular wall was placed or not placed.

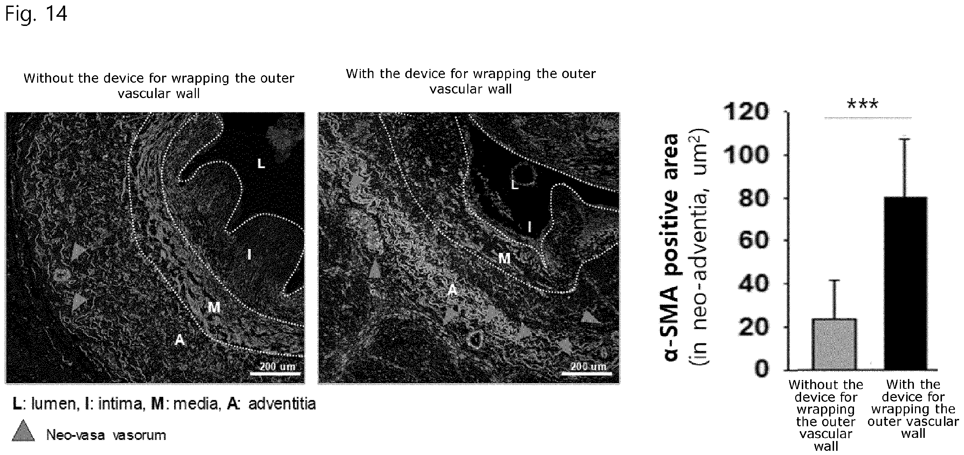

FIG. 14 illustrates confocal laser scanning microscopy results through immunostaining of Flk-1 and α-SMA and its quantitative graph in order to investigate the migration pattern of smooth muscle cells and neo-blood vessel formation according to whether or not a device for wrapping an outer vascular wall was placed.

### Mode for Carrying out the Invention

Hereinafter, the present invention will be described in more detail with reference to exemplary embodiments. These exemplary embodiments are provided only for the purpose of illustrating the present invention in more detail, and therefore, according to the present invention, it would be apparent to a person skilled in the art that these examples are not construed to limit the scope of the present disclosure.

### EXAMPLES

### Example 1: Compound synthesis

In a 3-neck round bottom flask, a magnetic bar was placed, and pentaerythritol (initiator, 0.5 mmol, Sigma Aldrich) and hydroquinone (inhibitor, HQ, 2.5 mmol, Sigma Aldrich) were added. The flask was capped, vacuumed for 10 minutes, and then purged with nitrogen gas at a rate of 50 cc/min. Purified ε-caprolactone (monomer, CL, 225 mmol, Sigma Aldrich) was injected into the flask by a 20 G syringe needle. The stirring was conducted at 180 rpm for 10 minutes at 110°C. Glycidyl methacrylate (monomer, GMA, 25 mmol, Sigma Aldrich) was injected by a 20 G syringe needle. Ten minutes after the injection of glycidyl methacrylate, 1,5,7-triazabicyclo[4.4.0]dec-5-ene (TBD, catalyst, 2.5 mmol, Sigma Aldrich) dissolved in 1 mL of acetonitrile (ACN, Sigma Aldrich) was injected by a 20 G syringe needle (2.5 mmol TBD/1 mL ACN). Thereafter, the mixture in the flask was reacted at 110°C for 6 hours. The final reaction product was dissolved in 15 mL of chloroform (Daejung chemicals & metals CO., LTD., Korea) and then precipitated in 400 mL of cold ethyl ether (Daejung chemicals & metals CO., LTD., Korea) at 4°C. The precipitate thus obtained was filtered and then dried under vacuum.

### Comparative Example 1: Control compound synthesis

In a 3-neck round bottom flask, a magnetic bar was placed, and 1,6-hexanediol (initiator, 0.5 mmol, Sigma Aldrich) and hydroquinone (inhibitor, HQ, 1 mmol, Sigma Aldrich) were added. The flask was capped, vacuumed for 10 minutes, and then purged with nitrogen gas at a rate of 50 cc/min. Purified ε-caprolactone (monomer, CL, 90 mmol, Sigma Aldrich) was injected into the flask by a 20 G syringe needle. The stirring was conducted at 180 rpm for 10 minutes at 110°C. Glycidyl methacrylate (monomer, GMA, 10 mmol, Sigma Aldrich) was injected by a 20 G syringe needle. Ten minutes after the injection of glycidyl methacrylate, 1,5,7-triazabicyclo[4.4.0]dec-5-ene (TBD, catalyst, 2.5 mmol, Sigma Aldrich) dissolved in 1 mL of acetonitrile (ACN, Sigma Aldrich) was injected by a 20 G syringe needle (1 mmol TBD/1 mL ACN). Thereafter, the mixture in the flask was reacted at 110°C for 6 hours. The final reaction product was dissolved in 15 mL of chloroform (Daejung chemicals & metals CO., LTD., Korea) and then precipitated in 400 mL of cold ethyl ether (Daejung chemicals & metals CO., LTD., Korea) at 4°C. The precipitate thus obtained was filtered and then dried under vacuum.

The raw material mixing for copolymer synthesis is shown in Table 1 below.

**TABLE 1**

| | HD (mmol) | Pentaerythrit ol (mmol) | TBD (mmol) | HQ (mmol ) | CL (mmol ) | GMA (mmol) |
|---|---|---|---|---|---|---|
| Linear PCL-PGMA | 0.5 | - | 1 | 1 | 90 | 10 |
| 4-arm PCL-PGMA | - | 0.5 | 2.5 | 2.5 | 225 | 25 |

1,6-Hexanediol (HD) and pentaerythritol were used as an initiator; 1,5,7-triazabicyclo[4.4.0]dec-5-ene (TBD) was used as a catalyst; hydroquinone (HQ) was used as an inhibitor; and ε-caprolactone (CL) and glycidyl methacrylate (GMA) were used as monomers.

### Example 2: Compound crosslinking

Each polymer was dissolved in N-methyl-2-pyrrolidone (NMP, Sigma Aldrich) at a ratio of 100 w/v%. Irgacure 2959 (photo-initiator, Sigma Aldrich) was added to the solution at a ratio of 0.1 w/v%. Thereafter, the solution was crosslinked (265 mW/cm², 200 s) in the form of a film by using a UV lamp (Omnicure S2000, Lumen Dynamics Group Inc., Canada).

### Example 3: Structure analysis (NMR measurement)

Structure analysis was conducted using H¹-NMR (AVANCE III HD 400, Bruker Biospin, USA) along with each polymer and Chloroform-D (Sigma Aldrich) at a concentration of 10 mg/Ml. The NMR measurement results are shown in FIG. 2.

PCL peaks were confirmed at δ=4.10[m, -OCH₂, (E)], 2.41[m, -CH, (A)], 1.74[m, -CH₂, (B and D)], and 1.45 [m, -CH₂, (C)], and PGMA peaks were confirmed at δ=6.13 [s, =CH₂, (G2)], 5.58 [s, =CH₂, (G1)], and 1.97 [s, -CH₃, (F)], indicating that linear PCL-PGMA(A) and 4-arm PCL-PGMA(B) were synthesized.

### Example 4: Molecular weight analysis (GPC)

Example 1-1 of PCL-co-PGMA in Korean Patent Registration No. 10-1906472 was used as a comparative example.

Molecular weight analysis was conducted using the PLgel 5 um MIXED-D column (Agilent Technologies Inc., USA) of gel permeation chromatography (GPC) along with each polymer and tetrahydrofuran (THF, J. T. Baker) at a concentration of 5 mg/mL. THF was used as an eluent, and the flow rate and the column temperature were 1 mL/min and 40°C, respectively. The standard curve was obtained using polystyrene (PS).

The molecular weight analysis results are shown in FIG. 3 and Table 2.

**TABLE 2**

| Polymer | Mn (Da) | Mw (Da) | PDI |
|---|---|---|---|
| Comparative example (Linear PCL-PGMA) | 8,976 | 13,418 | 1.49 |
| Example (4-arm PCL-PGMA) | 11,381 | 15,708 | 1.38 |

As shown in FIG. 3, the peak of the example was observed at a longer retention time than the peak of the comparative example, indicating a larger molecular weight. The GPC data measured to identify the molecular weight of the synthesized polymers are shown in Table 2. Compared with the comparative example, the example showed a higher molecular weight by about 2,300 Da and a smaller PDI value. Compared with the comparative example, the example showed a PDI value closer to 1, indicating a more uniform polymer chain size.

### Example 5: Differential scanning calorimetry (DSC)

Thermal characteristics were measured using a differential scanning calorimeter (DSC, Discovery DSC25, TA instrument Inc., USA). The sample was subjected to measurement at a rate of 10°C/min from -80°C to 150°C.

The DSC measurement Data before the crosslinking of the synthesized polymers are shown in Table 3.

**TABLE 3**

| Polymer | Tm, 1 (°C) | Tm, 2 (°C) | Hm (J/g) | Tc (°C) | Hc (J/g) |
|---|---|---|---|---|---|
| Comparative Example 1 (PCL) | 53.04 | - | 79.41 | 28.49 | 80.42 |
| Comparative Example 2 (Linear PCL-PGMA) | 46.08 | 51.18 | 72.61 | 21.3 | 70.93 |
| Example (4-arm PCL-PGMA) | 43.60 | 48.98 | 63.07 | 18.15 | 64.43 |

The reduction in crystallinity of a crystalline polymer results in reductions in the melting point (Tm), melting enthalpy (Hm), crystallization temperature (Tc), and crystallization enthalpy (Hc). When PGMA randomly bound to the crystalline polymer PCL, PGMA interfered with the formation of PCL crystals to reduce crystallinity thereof, so that compared with comparative example 1, comparative example 2 and the example showed reductions in the melting point (Tm), melting enthalpy (Hm), crystallization temperature (Tc), and crystallization enthalpy (Hc). In addition, in the comparison between comparative example 2 and the example, the example having a 4-arm structure was less crystallized than comparative example 2 having a linear structure, and such a structural difference resulted in lower melting and crystallization temperature. The example showed lower melting and crystallization temperatures and thus could be recovered to its shape at a lower temperature after crosslinking.

The DSC measurement results before and after the crosslinking of the synthesized polymer are shown in Table 4.

**TABLE 4**

| Polymer | Crosslinking | Tm (°C) | Hm (J/g) | Tc (°C) | Hc (J/g) |
|---|---|---|---|---|---|
| Comparative Example (PCL) | - | 53.04 | 79.41 | 28.49 | 80.42 |
| Example (4-arm PCL-PGMA) | Before crosslinking | 43.60 / 48.98 | 63.07 | 18.15 | 64.43 |
| | After crosslinking | 32.81 | 33.22 | -14.40 | 30.99 |

The DSC measurement results after the crosslinking of the synthesized linear PCL-PGMA and 4-arm PCL-PGMA are shown in Table 5 and FIG. 6.

**TABLE 5**

| Polymer | XTm (°C) | XHm (J/g) | XTc (°C) | XHc (J/g) |
|---|---|---|---|---|
| Comparative Example (Linear PCL-PGMA) | 39.40 | 44.39 | 1.20 | 43.84 |
| Example (4-arm PCL-PGMA) | 32.81 | 33.22 | -14.40 | 30.99 |

As for the crosslinking of a polymer, the acryloyl group of PGMA acts as a crosslinking point to allow the polymer to have a network structure, and the branches linking chains in the network structure interfere with crystallizing, thereby reducing crystallinity and further reducing the Tm value after crosslinking. The example had a 4-arm structure and thus had a reduced crystallinity as shown in Table 3, resulting in reductions in the melting point (Tm), melting enthalpy (Hm), crystallization temperature (Tc), and crystallization enthalpy (Hc), which were further reduced after crosslinking. The polymer compound of the present invention had reduced crystallinity and thus had a lower Tm value by about 6°C compared with the linear polymer, thereby recovering to its shape at a lower temperature and increasing the recovery rate to its original shape at the body temperature. Since the body temperature is higher than the Tm value of the polymer compound of the present invention, the polymer compound was almost a rubbery state in the body, and therefore, the insertion of the polymer compound into the body can reduce a foreign-object sensation.

### Example 6: Dynamic mechanical analysis (DMA)

Each sample crosslinked in a film form (W: 5 mm × L: 45 mm × T: 0.45 mm) was measured for mechanical properties by using dynamic mechanical analysis (DMA, Discovery DMA850, TA instruments Inc., USA). The sample was subjected to a tensile test at the body temperature 37°C at a rate of 10%/min to 190% strain. To measure the Young's modulus over temperature over time, the tensile test was conducted at 25°C (room temperature) and 37°C (body temperature) up to 190% strain at a rate of 10%/min, respectively.

The test results for tensile stress are shown in Table 6 and FIG. 7.

**TABLE 6**

| Polymer | Young's modulus (MPa) | Stress (MPa) | Max. strain (%) |
|---|---|---|---|
| Comparative Example (Linear PCL-PGMA) | 22.06±1.12 | 2.03±0.04 | 121.31±10.31 |
| Example (4-arm PCL-PGMA) | 10.00±1.79 | 1.67±0.04 | More than 190% |

Since the difference in Young's modulus value at 37°C between the comparative example and the example was 12 MPa or more, the example can more easily deform the shape than the comparative example. When tensioned with strain 190%, the comparative example was fractured at an average of 121.31%, but the example was not fractured even at 190%. Therefore, from the tensile test results at 37°C, the example has greater rubbery properties and showed a higher elongation than the comparative example.

### Example 7: Shape recovery ability

Each crosslinked film was placed in water at 55°C, deformed, and then fixed with liquid nitrogen (temporary shape), and thereafter, water having a temperature of a Tm value or higher was added to investigate its shape recovery. As a result of investigating the shape recovery ability, the 4-arm shape memory polymer had a shape recovery ability (see FIG. 8).

### Example 8: Prevention of stenosis of arteriovenous fistula model (side-to-side) using mammal

### 8-1. Preparation of arteriovenous fistula model

An incision of approximately 0.5 cm was made on the side of the femoral vein and the side of the femoral artery of a beagle dog weighing 8-10 kg, and the incised portions were allowed to face each other and subjected to side-to-side anastomosis using a suture, thereby constructing a vein to artery graft model. A temporary shape device for wrapping a outer vascular wall was placed at the blood vessel including the anastomosis site and treated with saline at 40°C to recover to its existing tube shape, thereby wrapping the blood vessels at the anastomosis site (see FIG. 12).

### 8-2. Blood vessel stenosis level and organ patency rate

The condition and size of blood vessels were investigated through histological analysis (H&E staining) of the vein graft according to whether or not a device for wrapping an outer vascular wall was placed.

As a result, the group without the placement of a device for wrapping an outer vascular wall showed that the vein was blocked or the neointima formation progressed significantly, resulting in poor patency, but the group with the placement of a device for wrapping an outer vascular wall showed that the neointima formation hardly progressed, indicating good patency (see FIG. 13).

These results established that the device for wrapping an outer vascular wall of the present invention suppressed the vortex formation caused by a difference in blood flow between the artery and the vein, thereby preventing blood vessel stenosis and improving organ potency rates.

### 8-3. Smooth muscle cell migration pattern

The smooth muscle cell migration pattern was investigated by immunostaining of an endothelial cell marker (Flk-1) and smooth muscle cell marker (alpha smooth muscle actin, α-SMA).

As a result, the group without the placement of a device for wrapping an outer vascular wall showed that the migration of smooth muscle cells to the intima was promoted to lead to neointima formation, but the group with the placement of a device for wrapping an outer vascular wall showed that smooth muscle cells migrate in a direction of the adventitia to inhibit neointima formation, leading to the formation of neo-adventitia (see FIG. 14).

These results established that the device for wrapping an outer vascular wall of the present invention induced smooth muscle cells of the media of a blood vessel in a direction of the adventitia, thereby preventing stenosis inside the blood vessel and reinforcing the vein with relatively weaker properties than the artery.

### 8-4. Induction of neo-vasa vasorum formation outside vein graft

The neo-blood vessel formation was investigated by immunostaining of an endothelial cell marker (Flk-1) and smooth muscle cell marker (alpha smooth muscle actin, α-SMA) .

As a result, a comparatively large amount of neo-blood vessels was formed in the adventitia in the group with the placement of a device for wrapping an outer vascular wall (see FIG. 14). During the construction of a vein-to-artery graft model, the stripping of the vein causes adventitia damage and vasa vasorum loss, thereby inducing a hypoxic state in the blood vessel wall, and this state causes an imbalance in the supply of nutrients and oxygen in the blood vessel to promote the mitigation of smooth muscle cells (SMCs) to the intima, causing neointimal hyperplasia and increasing the likelihood of blood vessel stenosis. Therefore, it is necessary to induce the formation of neo-vasa vasorum on the blood vessel wall for the smooth supply of nutrients and oxygen. It was established that the use of the device for wrapping an outer vascular wall of the present invention induced a light inflammatory response around the blood vessel, thereby inducing the formation of neo-blood vessels, and the formed neo-blood vessels act as a vasa vasorum to supply nutrients and oxygen and thus can prevent the development of neointimal hyperplasia and the occurrence of blood vessel stenosis.

### Industrial Applicability

The present invention relates to a shape memory polymer capable of recovering to its original shape from its deformed shape by the body temperature in the body.

## Claims

1. A shape memory polymer comprising a compound represented by Chemical Formula 1 below: 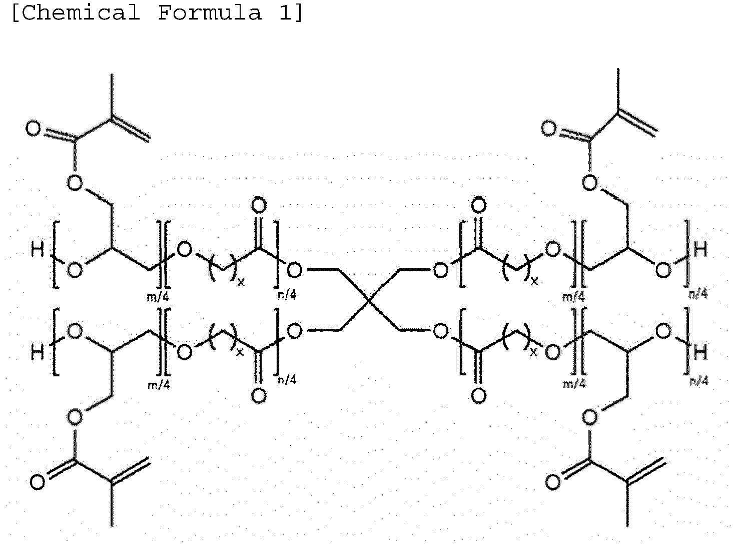 wherein,
x is an integer of 1 to 20;
m and n represent the mole % of repeating units, respectively; and
m + n is 100 and m is 80 to 96.

2. The memory shape polymer according to claim 1, wherein x is an integer of 2 to 10.

3. The memory shape polymer according to claim 1, wherein m is 92 to 96.

4. A device for wrapping an outer vascular wall, the device comprising the shape memory polymer of any one according to claims 1 to 3.

5. The device according to claim 4, wherein the outer vascular wall is an outer vascular wall at a blood vessel anastomosis site.

6. The device according to claim 5, wherein the blood vessel anastomosis site is an anastomosis site between two different blood vessels selected from a vein, an artery, and an artificial blood vessel.

7. The device according to claim 6, wherein the blood vessel anastomosis site is a vein-artery anastomosis site.

8. The device according to claim 6, wherein the blood vessel anastomosis site is a vein-artificial blood vessel anastomosis site or an artificial blood vessel-artery anastomosis.

9. The device according to claim 4, which has an original shape or a temporary shape, wherein the original shape is in a hollow cylindrical tube shape or a hollow Y-tube shape, one side of which is cut in a lengthwise direction to enable a blood vessel to be inserted into a hollow space,
wherein the temporary shape is maintainable at room temperature and is in a curved surface shape or a planar surface shape with both ends formed by the cutting being separated from each other, and
wherein when applied to an outer vascular wall, the temporary shape is bent in a direction of increasing the curvature to wrap the outer vascular wall, thereby recovering to the original shape.

10. A method for manufacturing a device for wrapping an outer vascular wall, the method comprising:
(a) photo-crosslinking a mixture of the shape memory polymer according to any one of claims 1 to 3 and a photo-initiator to a hollow cylindrical tube shape or a hollow Y-tube shape to prepare a tube-type device;
(b) cutting one side of the tube-type device in a length direction parallel to the central axis of a hollow space formed in the cylindrical tube or Y-tube; and
(c) inducing the tube-type device with one side cut, into a temporary shape allowing the insertion of a blood vessel under a temperature condition exceeding the body temperature and then fixing the tube-type device by cooling at a temperature lower than room temperature.

11. The method according to claim 10, wherein the mixture of the shape memory polymer and the photo-initiator in step (a) further contains a porogen.

12. The method according to claim 11, wherein the porogen is at least one selected from the group consisting of gelatin, sodium chloride, sodium bicarbonate, ammonium bicarbonate, polyethylene glycol, and hexane.

13. The method according to claim 10, wherein the photo-crosslinking in step (a) is performed by a 3D printer such that pores are formed.

14. The method according to claim 13, wherein the 3D printer is driven in any one manner selected from stereo lithography apparatus (SLA), digital light processing (DLP), and photopolymer jetting technology (PolyJet).

15. The method according to claim 10, wherein the temperature condition exceeding the body temperature is 42°C to 65°C.

16. The method according to claim 10, further comprising an additional porosity providing step after any one of steps (a) to (c).
